Europäisches Patentamt

European Patent Office

Office européen des brevets

(11) Publication number: 0 493 946 A2

# EUROPEAN PATENT APPLICATION

(21) Application number: 91311858.4

(22) Date of filing: 20.12.91

(51) Int. Cl.5: G01N 33/38

(30) Priority: 31.12.90 US 635980

(43) Date of publication of application:
08.07.92 Bulletin 92/28

(84) Designated Contracting States:
DE FR GB IT

(71) Applicant: WESTINGHOUSE ELECTRIC
CORPORATION
Westinghouse Building Gateway Center
Pittsburgh Pennsylvania 15222(US)

(72) Inventor: Clark, William Guy, Jr.
4215 Colonial Drive
Monroeville, PA 15668(US)
Inventor: Saunderse, Howard Edward
11 Carmel Court
Pittsburgh, PA 15221(US)
Inventor: Shannon, Robert Edward
28 Pheasant Road Run
Export, PA 15632(US)
Inventor: Sadhir, Rajender Kumar
5053 Impala Drive
Pittsburgh, PA 15239(US)

(74) Representative: van Berlyn, Ronald Gilbert
23, Centre Heights
London, NW3 6JG(GB)

(54) Method for monitoring the admixing of fluent materials.

(57) A method for monitoring the admixing of first and second fluent materials which ultimately harden into a solid structure. A selected quantity of a particulate tagging substance, whose presence is detectable by a fluctuating electromagnetic field is uniformly dispersed throughout the first fluent material and the first and second fluent materials are mixed together. The distribution of the detectable particulate tagging substance throughout the admixture is then monitored by transmitting the fluctuating magnetic field through it in order to determine the extent to which the materials are uniformly mixed together, as well as their relative ratios. When the admixture of fluent materials hardens into a solid structure, the uniform distribution of the particulate tagging substance throughout the structure advantageously allows it to be inspected for the presence of voids, cracks and discontinuities by the transmission of a fluctuating electromagnetic field through the structure.

FIG.1

This invention relates to a method for monitoring the admixing of fluent materials in order to determine not only the extent to which the materials are uniformly mixed together.

Sampling methods for monitoring the admixing of fluent materials to determine both the thoroughness of the mixing operation and the ratio of the fluent materials are known in the prior art. Such methods find application in such diverse fields as the mixing of concrete, the manufacture of plastic articles (where monomers and curing agents are mixed together to form hardened polymeric materials), the manufacture of pressed wood articles (where glue and sawdust are mixed together to form pressed wood), and even in the manufacture of explosives and rocket propellants, where organic binders are used to bind together small particles of reactive metal and oxidizers into certain shapes to achieve desired burning rates.

The prior art techniques for monitoring the progress of the mixing operation in such diverse industrial fields generally involves periodically capturing and inspecting a sample of the admixture during the mixing operation. For example, in the case of concrete mixing, a sample of the concrete being mixed is removed for the admixture and the water content of the concrete is indirectly determined by measuring the "slump" of the sample of concrete. If the slump measurements are too high (which indicates that the concrete mixture is too stiff), more water is added. Conversely, if the slump measurements are too low (indicating that the concrete mixture is too runny), additional cement, gravel and sand are added to the mixture.

Unfortunately, there are a number of drawbacks associated with such sampling methods. For example, of the periodic (as opposed to continuous) nature of the sampling, and the time it takes to perform each sampling operation, a significant amount of end product of unacceptable quality might be produced before such periodic sampling and inspection indicated a flaw in either the thoroughness of mixing, or the ratio of the fluent materials forming the end product. This problem is exacerbated in situations where the inspection operation does not yield the desired quality control information instantaneously, but only after significant amounts of time. For example, in the case of concrete, it takes a significant amount of time to perform the aforementioned "slump" test after a sample is withdrawn. Finally, such sampling methods cannot be used to test the structural integrity of the final product of the fluent materials after they harden into a solid shape. Any such structural tests will involve an entirely separate testing operation, which in many instances is not available in any practical form. For example, when concrete is poured into forms to create structural columns and beams, it is absolutely essential that the poured concrete be substantially free of air bubbles or other voids (known as "honeycombing" in this art) or the resulting structure may not be able to support its required load. Similarly, the presence of air bubbles or voids in finished plastic articles, or pressed wood products can seriously jeopardize the quality of these articles. In the case of explosives or rocket propellants, the presence of air bubbles or voids can render the end products highly dangerous, as the presence of any such bubbles or voids can dangerously accelerate the burning rate of the explosive or propellant matrix by increasing the combustible surface area of the matrix.

Clearly, there is a need for a method for continuously and accurately monitoring the admixing of fluent materials which ultimately harden into final products in order to determine both the thoroughness of the mixing step, and the ratio of the fluent materials within the admixture. Such a continuous monitoring method would provide the manufacturers of such diverse products as concrete, plastic articles, pressed wood articles and rocket propellants with a technique for insuring the final quality of their products, as such a method would provide continuous feedback signals which could instantaneously be used to correct any errors in either the thoroughness of mixing or the ratio of the materials used in the end products as soon as they occur. It would further be desirable if the monitoring method could be extended to inspect the structural integrity of the end products formed from the admixing operation so that voids, cracks, or discontinuities within the final products could be easily detected and the defective products either repaired or discarded.

Accordingly, the invention resides in a method for monitoring the admixing of first and second fluent materials which ultimately harden into a solid structure, characterized by uniformly dispersing a selected quantity of a particulate tagging substance into said first fluent material whose presence is detectable by radiant energy transmittable through said first fluent material; mixing said first and second fluent materials to form a mixture, and monitoring the distribution of said detectable particulate tagging substance throughout said mixture by transmitting said radiant energy through said mixture to determine the extent to which said materials are uniformly mixed together.

The method may further include the step of uniformly dispersing a selected quantity of a second particulate tagging substance whose presence is also detectable by radiant energy transmitted through the first and second fluent materials, but whose characteristics upon such detection are different from the detectable characteristics of the first particulate tagging substance. The use of such a second particulate tagging substance allows the operator of the method to immediately detect the presence of any voids in the admixture of the first and second fluent materials. Further, the method may include the steps of allowing the mixture to harden into a solid structure, and then inspecting the integrity of the structure by transmitting radiant energy through it in order to detect the presence of voids, cracks and discontinuities. The method of the invention finds particular application in the quality control of industrial processes where thoroughness of mixing, the ratio of the fluent materials admixed, and the presence or absence of voids in the admixture are important criteria. Advantageously, the same particulate tagging substances which allow the monitoring of the mixing operation may further be used to inspect the integrity of the resulting solid structure.

The particulate tagging substance may be particles of a ferromagnetic material, or a piezoelectric material, a material that effects the acoustical impedance of the fluent materials (such as fine particles of silicon dioxide), or particles that effect the optical properties of the fluent materials (such as vanadium dioxide, potassium di-hydrogen phosphate, and lithium niobate). The radiant energy used for the monitoring step of the method may include a fluctuating electromagnetic field (in the case of ferromagnetic material), a fluctuating electric field (in the case of piezoelectric material, ultrasound in the case of materials effecting the acoustical impedance of the fluent materials), or light (either visible or invisible) in the case of particles that effect the optical properties of the fluent materials.

The method of the invention may be applied to the mixing of common structure concrete, the mixing of Polysil(R) concrete, the mixing of urethane monomers with a curing agent of the type applied to the hulls of naval vessels, and the mixing of binding agents to explosive mixtures of metal particles and oxidizers in the manufacture of shaped charges used in military ordnance, as well as the mixing of glue and particulate wood in the manufacture of particle board. The method provides a convenient means of inspecting not only the structural integrity of the resulting structure (as may be the case with concrete or particle board), but also the presence of areas of disbonding in instances where the fluent structure hardens over a surface in order to bind the structure thereto (as in the case of polyurethane over the hull of a naval vessel).

In order that the invention can be more clearly understood, convenient embodiments thereof will now be described, by way of example, with reference to the accompanying drawings in which:

Figure 1 is a schematic view of a system for monitoring the admixing of two fluent materials according to a first embodiment, wherein only one of the two fluent materials mixed contains tagged particles;

Figure 2 is a perspective view which illustrates how the finished products that result from the admixture of the fluent materials illustrated in Figure 1 may be inspected by, for example, an eddy current probe in order to determine the presence of any bubbles or cracks within the product;

Figure 3 is a schematic view of a system for monitoring the admixing of fluent materials according to a second embodiment, wherein each of the fluent materials mixed includes a different type of tagging particle;

Figure 4 illustrates how a finished product created by the mixing system illustrated in Figure 3 might be inspected not only for bubbles, but for non-homogeneities in mixing, and for regions of disbonding when the finished product is a bonding coating;

Figure 5 is a graph illustrating how the eddy current response increases as a weight percentage of the content of the tagged particles (which in this example is particulate ferrite), and how this response varies at frequencies of 100 hertz, 500 hertz and 1 kilohertz, and

Figure 6 is a graph illustrating how the eddy current response varies as a function of the weight percentage of the tagged particles for a variety of fluent mediums, such as water, cement, plaster and adhesive.

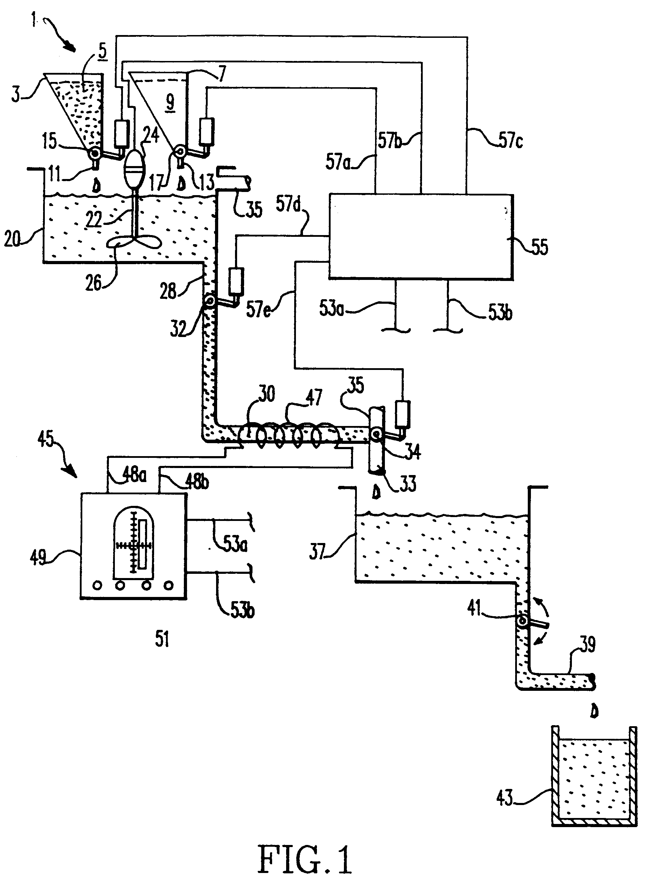

With reference now to Figure 1, wherein like components are designated by like numerals throughout all the several figures, system 1 comprises a hopper 3 filled with a first fluent material 5 into which a quantity of a particulate tagging substance has been dispersed (as is indicated by the dots), and another hopper 7 which contains untagged fluent material 9. In this particular example of the system 1, the tagged fluent material 5 is an aqueous solution of ferromagnetic iron lignosulfonate which contains about one percent by weight of very fine, spinel ferrite particles having diameters of about 50 to 150 Angstroms. Such a solution of ferromagnetic iron lignosulfonate is available from the Georgia Pacific Corporation, located in Bellingham, Washington. The untagged fluent material 9 is a dry concrete mixture of Portland cement, sand and gravel. While in this example the fluent materials 5 and 9 are water and concrete, both the system and method of the invention are applicable to mixtures of virtually any two fluent materials which ultimately harden into a solid structure.

Each of the hoppers 3,7 includes an outlet 11,13 through which the fluent materials 5,9 may flow, either continuously or intermittently. The flow of such materials through the outlets 11,13 is modulated by motor operated valves 15,17. The system 1 of the invention also has a mixing vessel 20 for receiving the fluent materials 5,9 which flow through the outlets 11,13 of the hoppers 3,7. An electric mixer 22 is provided for admixing the fluent materials 5,9 to form a homogeneous admixture of these materials. For this purpose, the mixer 22 includes a motor 24 which turns a propeller 26 by way of a shaft as shown to mix and agitate the fluent materials 5,9. The mixing vessel 20 has an outlet 28 for discharging the admixture of the fluent materials 5,9 created by the electric mixer 22.

The system 1 further includes an inspection and drain conduit 30 which conducts a flow of the admixture generated within the mixing vessel 20 and permits it to be inspected by a source of radiant energy capable of detecting both the concentration and distribution of the particulate tagging material dispersed throughout the admixture. To these ends, it is important that the walls of the inspection drain conduit 30 be formed from a material which is substantially transparent to whatever type of radiant energy is used to detect both the concentration and distribution of the tagged particulate material. In this example, since a fluctuating, high frequency electromagnetic field is used to detect both the concentration and the distribution of the ferrite particles distributed throughout the concrete mix created in mixing vessel 20, the walls of the inspection and drain conduit 30 are preferably formed from a plastic material such as polyvinyl chloride. Additionally, the inspection and drain conduit 30 should be dimensioned so that it easily accommodates whatever structural form the source of particle-detecting radiant energy may take. In the instant invention, since the source of the high frequency fluctuating electromagnetic field may conveniently be a simple electrical coil, the conduit 30 is tubular in shape with its outer diameter being less than the inner diameter of the coil used to generate the detecting radiant energy.

The upstream end of the inspection and drain conduit 30 is connected to the outlet 28 of the mixing vessel 20 through a motor operated discharge valve 32 as shown, while the downstream end of the conduit 30 includes both an outlet 35 which is disposed over a reservoir vessel 37, a motor operated diverter valve 34, and a recycling conduit 35 which is connected back to the mixing vessel 20. Under normal operating conditions, the admixture flowing through the conduit 30 will be discharged into the reservoir vessel 37. However, should the admixture prove defective, the diverter valve 34 will shift position to recycle the admixture back to the mixing vessel 20 via conduit 35 for correction.

The purpose of the reservoir vessel 37 is to hold an inventory of the admixture of the fluent materials 5,7 created within the mixing vessel 20 for final use in creating a finished product. Like the previously described mixing vessel 20, the reservoir vessel 37 also includes an outlet 39 whose output is modulated by means of a final discharge valve 41. In this example of the system 1 of the invention, the outlet 39 is disposed over a mold 43 which molds the fluent admixture created in the mixing vessel 20 to a final desired shape. The provision of the reservoir vessel 37 within the system 1 advantageously accommodates short-term variations in the rate at which an admixture is created within the mixing vessel 20 and the rate at which this admixture is applied to a final purpose, such as the creation of a solid structure within the mold 43.

System 1 further includes a tagged particle detector 45. In this example of the invention, the tagged particle detector 45 includes a sensing coil 47 looped around the inspection and drain conduit 30 as shown. The leads 48a,b of the sensing coil 47 are connected to a multi-frequency generator 49 of the type used in eddy current probe inspections. In the preferred embodiment, the multi-frequency generator 49 is preferably part of Miz-18 eddy current inspection system manufactured by Zetec located in Isaquah, Washington. Such commercially available inspection systems operate by generating and conducting a high frequency alternating current through a sensing coil such as coil 47 so that the coil radiates a fluctuating electromagnetic field, and further by measuring the amount of impedance that this electromagnetic field incurs as the result of electromagnetic coupling between the field and the ferrite tagging particles distributed throughout the admixture flowing through the conduit 30. From the magnitude and location of this measurable impedance, both the concentration and the distribution of the ferrite tagging particles within the admixture flowing through the conduit 30 may be measured. From these measurements, both the relative concentration of the fluent materials 5,9 and the thoroughness by which they were mixed within the vessel 20 may also be inferred, and displayed on a CRT readout screen 51. The multi-frequency generator measurements of both the concentration and distribution of the ferrite tagging particles may be reduced to electrical signals which may be transmitted outside of the multi-frequency generator 49 via output cables 53a,b.

Finally, system 1 includes a microprocessor control circuit 55 whose input is connected to the output cables 53a,b of the multi-frequency generator 49, and whose output is connected to control cables 57a,b,c,d,e which are connected to the motor operated valve of the hopper 5, the electric motor 24 of the mixer 22, the motor operated valve 17 of the hopper 7, and the motor operated discharge valve 32 disposed on the outlet 28 of the mixing vessel 20, and the motor operated diverter valve 34 disposed upstream of the outlet 33 of the inspection and drain conduit 30. One of the functions of the microprocessor control circuit 55 is to modulate the flow of both tagged and untagged fluent material 5,9 from the hoppers 3,7 into the mixing vessel 20 so that the ratio of these materials 5,9 in the final admixture flowing out through the outlet 28 continuously remains within a range corresponding to a selected manufacturing tolerance. Another function of the microprocessor control circuit 55 is to operate the electric mixer 22 at a speed and over a sufficient length of time with the discharge valve 32 closed such that the tagged and untagged fluent materials 5,9 are thoroughly and homogeneously admixed at the time that the admixture is finally discharged through the motor operated discharge valve 32. In the event that the signal generated by the tagged particle detector 45 indicate that the ratio of the mixture is no longer within the preselected range or that the fluent materials 5,9 are insufficiently mixed, the microprocessor control circuit 55 changes the position of the diverter valve 34 to recycle the defective admixture back to the mixing vessel 20 via conduit 35 for correction, either by adding more or less of the fluent materials 5,9, or by varying the speed of the mixer motor 24, or by increasing the mixing time within the vessel 20 by keeping the discharge valve 32 closed for longer lengths of time. The continuous monitoring of both the concentration and distribution of the ferrite tagging particle in the admixture by the tagged particle detector 45, and the continuous transmission of this information into the input of the microprocessor control circuit via cables 53a,b provide in effect a continuous feedback signal to the microprocess or control circuit 55 which allows it to quickly and advantageously manipulate the motor operated valves 15,17 of the hoppers 3,7, the motor 24 of the electric mixer 22, the discharge valve 32 of the mixing vessel 20 and the diverter valve 34 of the conduit outlet 33 to continuously and automatically create an admixture whose fluent components stay within a desired ratio and whose components are homogeneously intermixed to virtually any preselected degree of thoroughness before being discharged through conduit outlet 33 into the reservoir vessel 37.

While in this example of the tagged fluent material 5 is water that has been tagged with fine particles of spinel ferrite and the untagged fluent material 9 is a dry concrete mixture, it should be noted that the invention is not confined to the use of these particular tagging particles in fluent materials. For example, the tagging particles may be formed from a piezo-electric material such as quartz, or a material which measurably changes the acoustic impedance of the surrounding fluent material (which again may be fine particles of quartz). If piezo-electric or acoustical-impedance type tagging particles are used, then the tagged particle detector 45 should radiate either a fluctuating electric field or ultrasound through the inspection and drain conduit 30. Alternatively, the tagging particles may be formed from a substance that effects the optical properties of the fluent materials with which it is admixed, such as particles of vanadium dioxide, potassium di-hydrogen phosphate, and lithium niobate. In such an instance, the tagged particle detector 45 should generate a form of light (which may be either visible or invisible) which easily penetrates the fluent materials forming the admixture and the material forming the walls of the inspection and drain conduit 30, but which is absorbed by (or otherwise measurably interacts with) the particulate tagging substance. Other examples of admixtures of fluent materials which can be effectively monitored by the system 1 of the invention include the admixing of monomers with curing agents to create polymer plastics, the admixture of glue with particulate wood to form particle board, the admixture of a hardenable resin with fillers of silica and alumina to form Polysil(R) concrete, and the admixture of organic binding agents with fine particles of active metals and oxidizers to form shaped charges used in explosives and rocket propellants that burn at controlled rates.

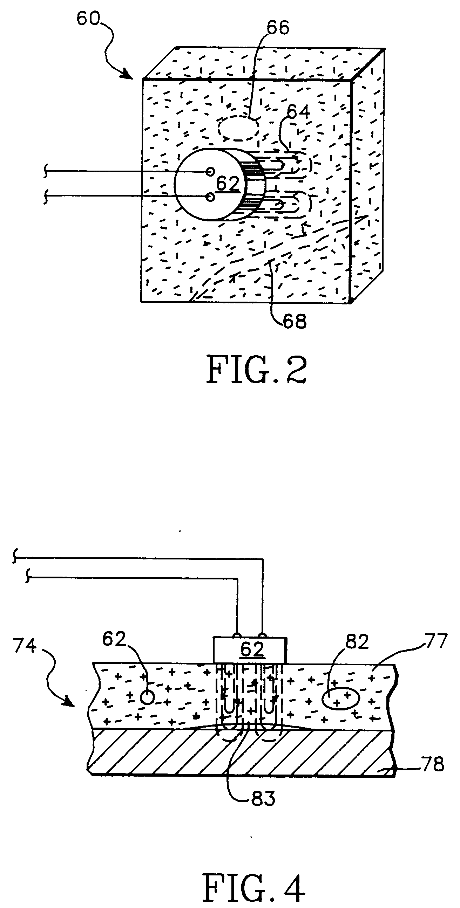

Figure 2 illustrates how the structural integrity of a finished product 60 formed from the hardened admixture created in the mixing vessel 20 might be inspected by a "pancake" type eddy current probe assembly 62. Such probe assemblies 62 include an annular coil (not shown) which radiates a fluctuating magnetic field 64 capable of penetrating deeply into a non-metallic solid structure. For example, such a finished product 60 may be inspected by a means of a model SPO air-core "pancake" eddy current probe that is connected to a model NDT No. 25 L programmable test instrument, both of which are manufactured by Nortec located in Kennewick, Washington. The scanning may be performed by means of a model STC 460 X-Y scanner manufactured by the Daedal Corporation located in Harrison City, Pennsylvania, and the results may be interpreted by means of a model LSI 11/23 computer manufactured by the Digital Equipment Corporation located in Maynard, Massachusetts, and displayed on a model 4695 plotter manufacturer by the Tektronix Corporation located in Beaverton, Oregon. Such commercially available instrumentation would be easily capable of detecting any voids or "bubbles" 66 present within the walls of the finished product 60 that might seriously jeopardize its ability to perform its intended function (i.e., such as the bearing of a specified load in the case of concrete formed into the structural component of a building). Such an inspection may also be used to detect the presence of other discontinuities, such as cracks 68. Fortunately, in the case where the spinel ferrite particles present in lignosulfonate are used as the tagging particles, the extremely small size of these particles allows an extremely large number of such particles to be distributed throughout the solid matrix resulting from the hardening of the fluent materials containing such ferrite tagging particles, even when these particles form only a small weight percent (such as one percent) of the admixture. The large number of particles present in even a very small weight percent in turn allows a very fine resolution picture of the internal structure of the finished product 60 to be made by means of the aforementioned commercially-available eddy current probes and related scanning equipment. A further advantage of the extremely small particle size of the spinel ferrite contained within lignosulfonate is that it forms a colloidal suspension in even relatively non-viscous liquids, such as water, and hence will not tend to either settle to the bottom of the admixture, or buoyantly float to the top of it.

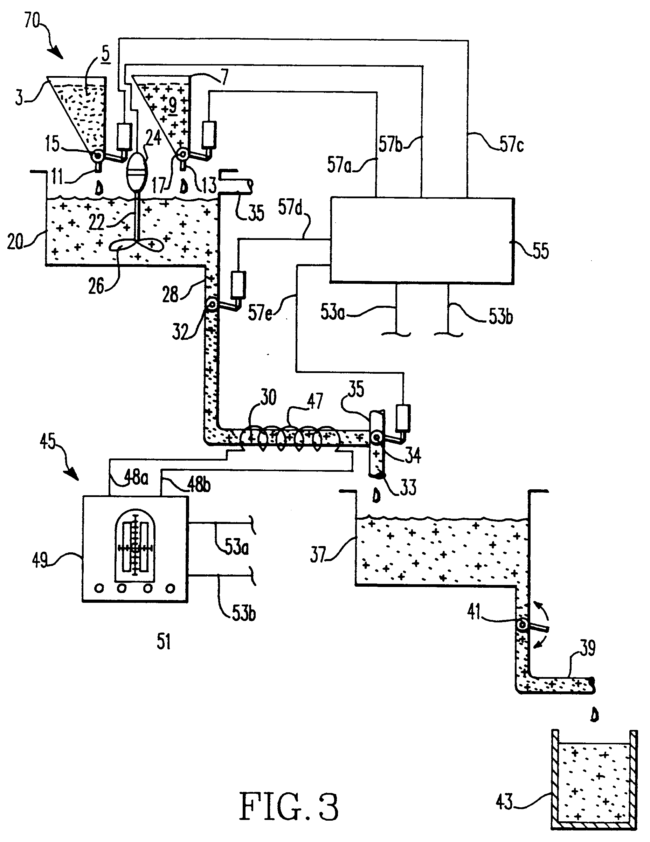

Figure 3 illustrates a modified system 70 which is exactly the same in all respects as the previously discussed system 1 of the invention, with the exception that a second type of particulate tagging substance (schematically illustrated by small crosses) is distributed throughout the second fluent material 72. In system 70, the particulate tagging material uniformly distributed through the second fluent material 72 is a spinel ferrite having measurably different absorption characteristics with respect to the fluctuating electromagnetic field emanated by the sensing coil 47 of the tagged particle detector 45. In operation, the multi-frequency generator 49 of the tagged particle detector 45 would alternately generate alternating currents having different frequencies so that the frequency of the alternating magnetic field generated by the sensing coil 47 would periodically change from a higher frequency to a lower one and then back to the higher frequency. By rapidly multiplexing the frequency of the alternating current it generates, the multifrequency generator 49 of the tagged particle detector 45 could be made to "see" two separate images of the fluent material flowing through the inspection and drain conduit 30, as is schematically illustrated on the CRT readout screen 51. Such alternate sensing of the different tagging particles used in the first and second fluent materials 5,72 allows the microprocessor control circuit 55 of this embodiment 70 of the system to unambiguously interpret a space in the admixture which is entirely devoid of either type of tagging particle as a bubble or air space in the admixture flow. By contrast, in the first described system 1, such a space might indicate an area of incomplete mixing that is entirely filled up by the untagged fluent material 9. System 70 advantageously allows the microprocessor control circuit 55 to correctly interpret areas in the admixture wherein only one of the two types of tagging particles is present as being an area wherein insufficient mixing has taken place. In such instances, the microprocessor control circuit 55 may take corrective action by either increasing the speed of the electric motor 24 of the mixer 22, or by increasing the time of the mixing operation within the mixing vessel 20 by keeping the motor operated discharge valve 32 closed for longer lengths of time between discharges. Hence, the use of separately detectable types of tagging particles in the two fluent materials forming the admixture within the mixing vessel 20 creates new information which may be advantageously used for tighter and more comprehensive quality control.

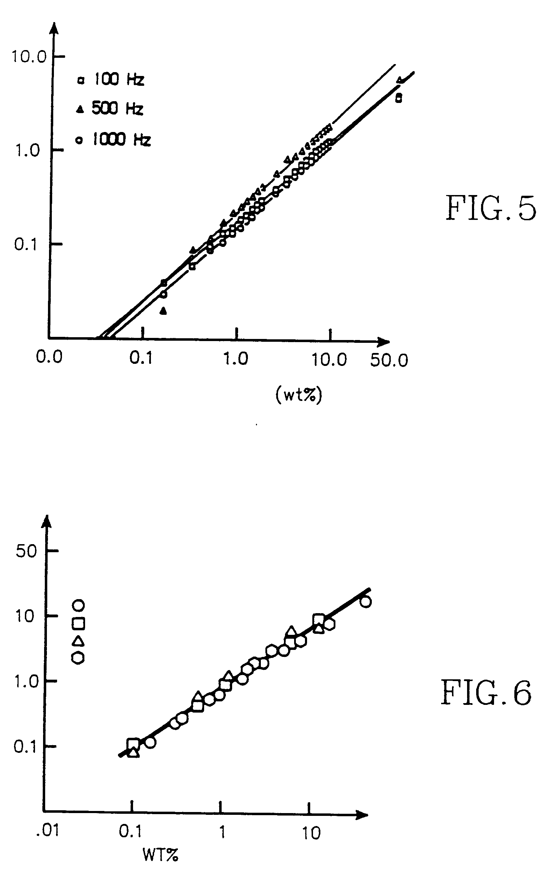

Figure 4 illustrates how the presence of two different types of tagging particles in the finished product 74 created by the alternate system 70 may be used to effect an even more detailed inspection of such products. In this particular example, the finished product 74 is a coating of polyurethane 77 which is applied over the hull 78 of a naval vessel to muffle the reflection of enemy sonar. When this coating 77 is inspected by means of the previously-described eddy current probe assembly 62 (which in this case is operated by multiplexing different frequency alternating currents through the annular coil disposed in the probe head), the probe assembly 62 is capable of resolving the difference between a bubble 66 in which neither of the tagged particles is present, and an area 82 of incomplete admixing wherein only one type of tagged particle is predominantly present. Additionally, in this particular finished product 74, the probe assembly 62 is capable of detecting an area 83 of disbonding between the ship hull 78 and the sonar adsorbing plastic coating 77, as such an area 83 will, like the bubble 66, be characterized by the total absence of either type of tagged particle. The ability to unambiguously detect such unbonded areas 83, and to distinguish them from unmixed areas 83 is of particular importance with respect to sonar absorbing coatings 77, as a disbonded area 83 substantially impairs the ability of such coating to perform its intended function, while a small local area 82 of insufficient admixing of the components forming the polyurethane would not significantly effect such sonar absorbing characteristics.

Figure 5 is a graph illustrating how the response of the eddy current probe used in both the tagged particle detector 45 and in the probe assembly 62 illustrated in Figures 1 through 4 changes with respect to the content of the ferrite particles present in the admixture. As is evident from this graph, the eddy current response is essentially a linear function of the weight present of ferrite content within the admixture. Moreover, the eddy current response remains substantially unchanged over a fairly broad spectrum of alternating current frequencies ranging from 100 hertz, to 1 kilohertz.

Figure 6 illustrates how the eddy current response increases with the weight percent of ferrite in various fluent materials, including water, cement, plaster and a polymer adhesive (represented by circles, square, triangles and hexagons, respectively). Essentially, this graph indicates that the eddy current response increases linearly with the weight percent of ferrite in almost exactly the same manner for each of these four different types of fluent materials. While the data represented by the graphs of Figures 5 and 6 are by no means a complete picture, they do suggest that the eddy current response of a tagged admixture may be easily and simply predicted for a broad range of materials on the basis of the weight percent of the amount of spinel ferrite particles distributed throughout the admixture, and that the eddy current response will remain substantially unchanged over a broad range of detection frequencies.

The method of the invention may be understood in the context of the operation of either of the systems 1,70. In the first step of the method of the invention, a selected quantity of a particulate tagging substance (such as the fine spinel ferrite particles present in ferro-magnetic iron lignosulfonate) is dispersed throughout one of the fluent materials 5 of the admixture. The two fluent materials 5,9 are then admitted out of the outlets 11,13 of their respective hoppers 3,7 into the mixing vessel 20, and mixed by means of the mixer 22. After the mixer 22 has been operated for a long enough time to thoroughly admix the two fluent materials, the motor operated discharge valve 32 which is mounted over the outlet 28 of the mixing vessel 20 is opened to start a flow of the completed admixture to be conducted through the inspection and drain conduit 30.

At this juncture, the tagged particle detector 45 is actuated in order to determine both the concentration and the distribution of the tagged particles within the admixture. In both embodiments of the system 1,70, the tagged particle detector 45 includes a sensing coil 47 and a multi-frequency generator 49 for generating a high frequency alternating electromagnetic field which penetrates completely through the admixture flowing through the inspection and drain conduit 30. At this juncture in the method of the invention, the tagged particle detector 45 continuously generates electric signals indicative of both the concentration and distribution of the tagged particles, and transmits these signals to the microprocessor control circuit 55 via cables 53a,b. From this information, the microprocessor control circuit 55 infers both the ratio of each of the fluent material within the admixture flowing through the conduit 30, as well as the thoroughness of the mixing operation. The microprocessor control circuit continuously compares these readings with a preselected range of desired ratios, and a desired mixing level. Whenever the measured ratios and mixing level varies from these preselected ranges and values, the microprocessor control circuit 55 shunts the flow of admixture from the conduit outlet 33 to the recycle conduit 35 by changing the position of the diverter valve 34, adjusts the motor operated valves 15,17 of the fluent material hoppers 5,9, and/or adjusts the speed of the motor 24 of the electric mixer 22 and the position of the mixing vessel discharge valve 32 to bring these values back into conformance with the desired values. In the final step of the method of the invention, the structural integrity of the finished product 60,74 produced by the systems 1,70 may be inspected in the aforementioned manner by means of an eddy current probe assembly 62.

## Claims

1. A method for monitoring the admixing of first and second fluent materials which ultimately harden into a solid structure, characterized by: uniformly dispersing a selected quantity of a particulate tagging substance into said first fluent material whose presence is detectable by radiant energy transmittable through said first fluent material; mixing said first and second fluent materials to form a mixture, and monitoring the distribution of said detectable particulate tagging substance throughout said mixture by transmitting said radiant energy through said mixture to determine the extent to which said materials are uniformly mixed together.

2. A method according to claim 1, characterized by remixing the mixture if the fluent materials are not admixed to a desired extent.

3. A method according to claim 1 or 2, characterized by allowing the mixture to harden into a solid structure, and then inspecting the integrity of said structure by transmitting the radiant energy through said structure to detect the presence of voids, cracks and discontinuities.

4. A method according to claim 1, 2 or 3, characterized in that the detectable particulate tagging substance includes particles of ferromagnetic material, and the radiant energy is a fluctuating electromagnetic field.

5. A method according to claim 1, 2 or 3, characterized in that the detectable particulate substance includes particles of a piezo-electric material, and the radiant energy is a fluctuating electric field.

6. A method according to claim 1, 2 or 3, characterized in that the detectable particulate tagging substance includes particles of a material which effects the acoustical impedance of the first fluent material, and the radiant energy is ultrasound.

7. A method according to claim 6, characterized in that the detectable particulate tagging substance includes particles of quartz.

8. A method according to claim 1, 2 or 3, characterized in that the detectable particulate tagging substance includes particles of a material which effects the optical properties of the first fluent material, and the radiant energy is radiant electromagnetic energy.

9. A method according to claim 8, characterized in that the detectable particulate tagging substance comprises particles of at least one of vanadium dioxide, potassium di-hydrogen phosphate and lithium niobate.

10. A method according to any of claims 1 to 9, characterized by uniformly dispersing a selected quantity of a second particulate tagging substance whose presence is detectable by radiant energy transmitted through the second fluent material.

11. A method according to any of claims 1 to 10, characterized by applying the resulting mixture over a surface in order to bond the resulting solid structure to said surface, and then inspecting the interface between the solid structure and the surface by transmitting the radiant energy through said interface to detect the presence of any disbonding.

12. A method according to claim 11, characterized in that the first fluent material in unpolymerized urethane, the second fluent material is a curing agent for curing the urethane into solid polyurethane, and the surface is the exterior surface of the hull of a water vessel.

13. A method according to any of claims 1 to 10, characterized in that the first fluent material is water, and the second fluent material is a particulate concrete mix, and the monitoring of the distribution of the detectable particulate tagging material throughout the mixture is used both to determine the ratio of water to said particulate concrete mix, and to determine the thoroughness of the mixing of said water and concrete mix prior to the hardening of said mixture.

14. A method according to claim 13, characterized in that the distribution of the particulate tagging substance is continuously monitored in the mixture of water and particulate concrete to continuously measure the extent to which the water and concrete mix were admixed and the ratio of the water to the concrete mix.

15. A method according to claim 14, characterized in that the particulate ferromagnetic particles constitute from 0.5 to 10, preferably 0.5 to 3, weight percent of the water.

16. A method according to any of claims 1 to 10, characterized in that the first fluent material is a polymerizable resin, and the second fluent material is a particulate ceramic filler material.

17. A method according to any of claims 1 to 10, characterized in that the first fluent material is glue, and the second fluent material is particulate wood, and said solid structure is particle board.

18. A method according to any of claims 1 to 10, characterized in that the first fluent material is an organic binder, and the second fluent material is a mixture of reactive metal particles and oxidizer particles, and the structure is a solid ordinance propellant.

FIG.1

60

66

64

62

68

# FIG.2

74

62

62

82

77

78

83

# FIG.4

FIG.3

FIG.5

FIG.6